# EUROPEAN PATENT APPLICATION

(11) **EP 2 305 661 A1**
(43) Date of publication of application: **06.04.2011**
(21) Application number: 09171996.3
(22) Date of filing: 01.10.2009
(51) Int. Cl.: C07D 309/18

(54) **Process for the preparation of 2,3-dihydropyran**

(71) Applicant: Lonza Ltd., 4052 Basel (CH)
(72) Inventor: Künzle, Niklaus, CH-3938 Ausserberg (CH)
(74) Representative: Winter, Brandl, Fürniss, Hübner, Röss, Kaiser, Polte - Partnerschaft

(57) **Abstract**

The present invention relates to a process for producing 2,3-dihydropyran (DHP), comprising contacting tetrahydrofurfuryl alcohol (THFA) entrained in a carrier gas with a catalyst comprising aluminium oxide (Al₂O₃), wherein the carrier gas is water vapour.

## Description

The present invention relates to a process for the preparation of 2,3-dihydropyran (DHP).

2,3-Dihydropyran is an important building block in the synthesis of pharmaceutical compounds. Several processes for the preparation of DHP are described in the prior art.

US-A-2,976,299 discloses a process for producing DHP wherein vaporized tetrahydrofurfuryl alcohol (THFA) is passed through a catalyst comprising Al₂O₃ and TiO₂ followed by condensation of the obtained product. The vaporized THFA is entrained in a carrier gas such as nitrogen or hydrogen.

US-A-3,518,281 discloses a process for producing DHP by conversion of vaporized THFA at a temperature from 200 to 375 °C using η-Al₂O₃ as a catalyst. As above, the THFA may be entrained in a carrier gas such as nitrogen or argon when contacted with the catalyst.

If gases such as nitrogen are used, however, large amounts of gas are required which for economic reasons must be recollected and compressed again. In addition, considerable amounts of product are lost by removal with the nitrogen gas, as condensation is usually not sufficient for completely separating the products from the nitrogen gas. Moreover, the above processes suffer from the drawback that they require frequent regeneration of the catalyst. The regeneration is typically carried out at elevated temperatures in the presence of air or oxygen while the catalyst is still installed and thus resulting in interruption of the production.

The object of the present invention, therefore, is to provide a simple and cost efficient process for the production of DHP, wherein DHP is obtained in high yields, with low amounts of by-products and good recovery rates, and wherein the used catalyst has a long life and little regeneration is required.

This object has been achieved by the present invention, which provides a process for producing DHP, comprising contacting THFA entrained in a carrier gas with a catalyst comprising aluminium oxide (Al₂O₃), wherein the carrier gas is water vapour.

Scheme 1 below shows the conversion of THFA taking place in the presence of a solid phase Al₂O₃ catalyst to form DHP and H₂O, which typically takes place at elevated temperatures.

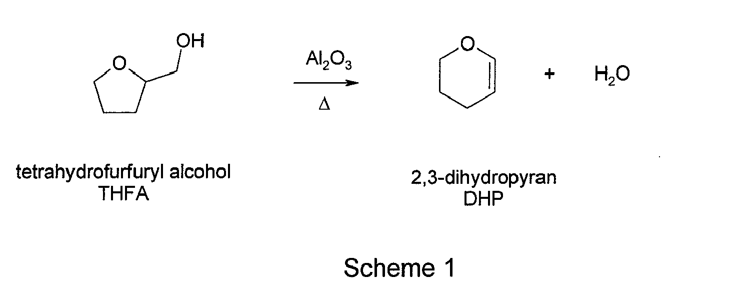

### Figures

Figure 1 shows a schematic representation of the production process of DHP according to the present invention with subsequent distillation.
Figure 2 shows the reactor temperature measured during a 4.5 day run of a production process according to the present invention.
Figures 3 to 7 show the contents of different compounds in the crude product during a 4.5 day run of a production process according to the present invention (measured by gas chromatography with FID detection, column Type DB-1701 of dimensions 10 m x 0.18 mm x 0.4 µm, and expressed as % area of the peak of the respective compound in the chromatogram what corresponds with the weight % of the respective compound in the crude product), wherein

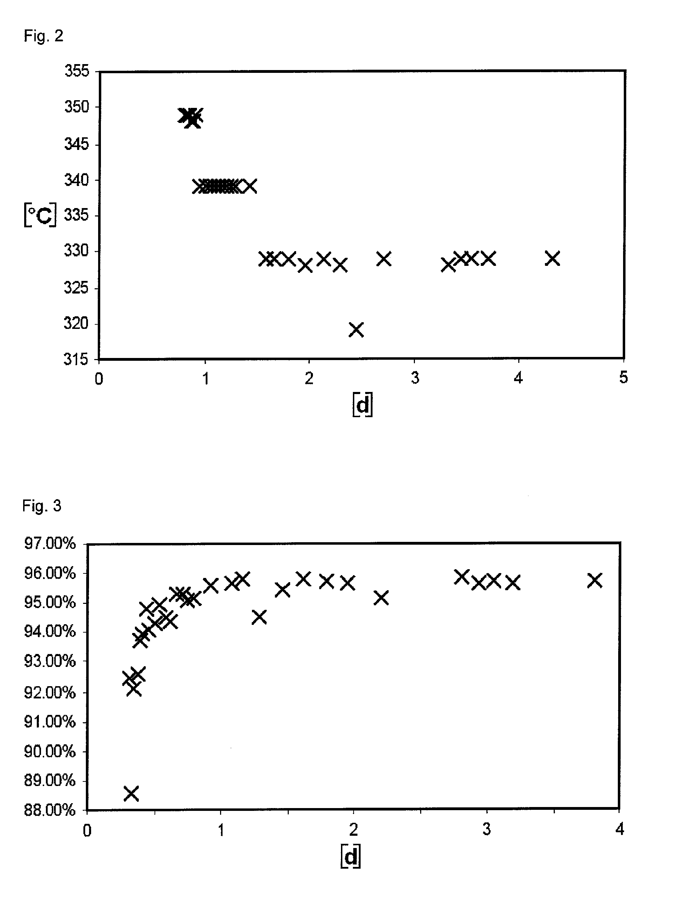

Figure 3 shows the content of generated DHP in % area vs. running time in days,

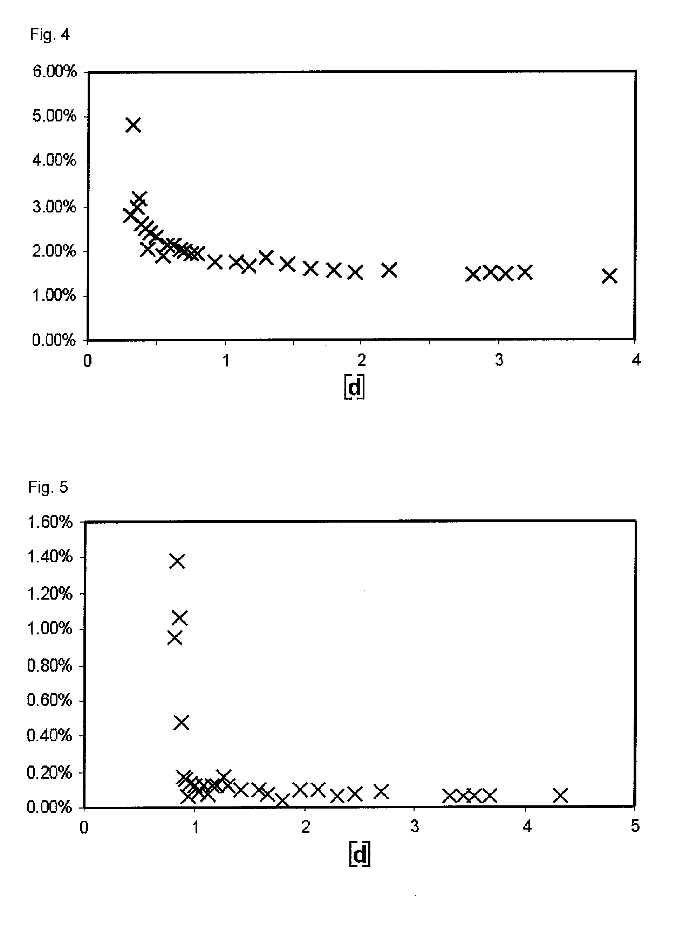

Figure 4 shows the content of the by-product THP in % area vs. running time in days,
Figure 5 shows the content of the by-product cyclopentanone in % area vs. running time in days,

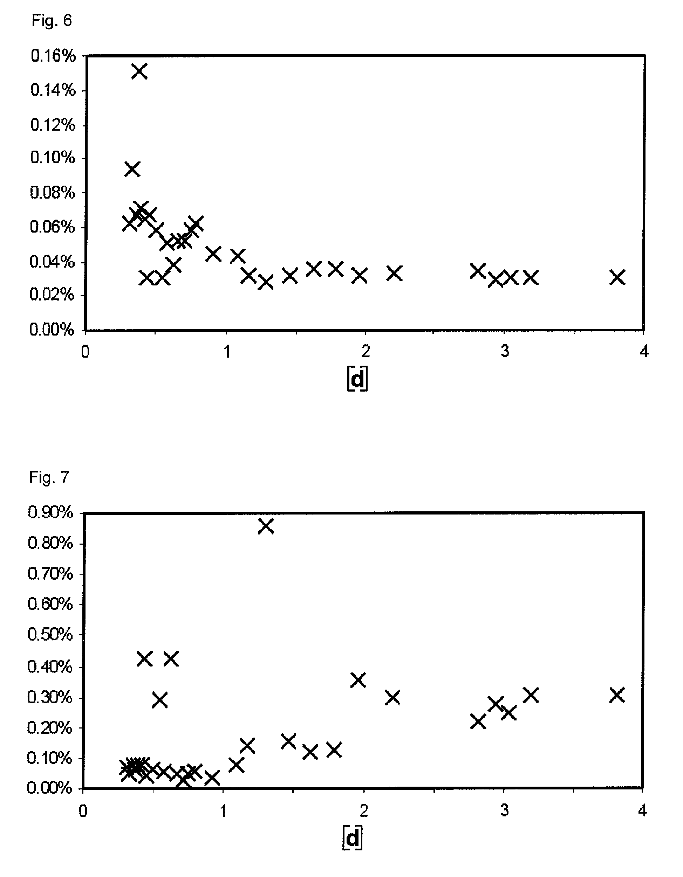

Figure 6 shows the content of the by-product acrolein in % area vs. running time in days, and
Figure 7 shows the content of unconverted THFA in % area vs. running time in days.

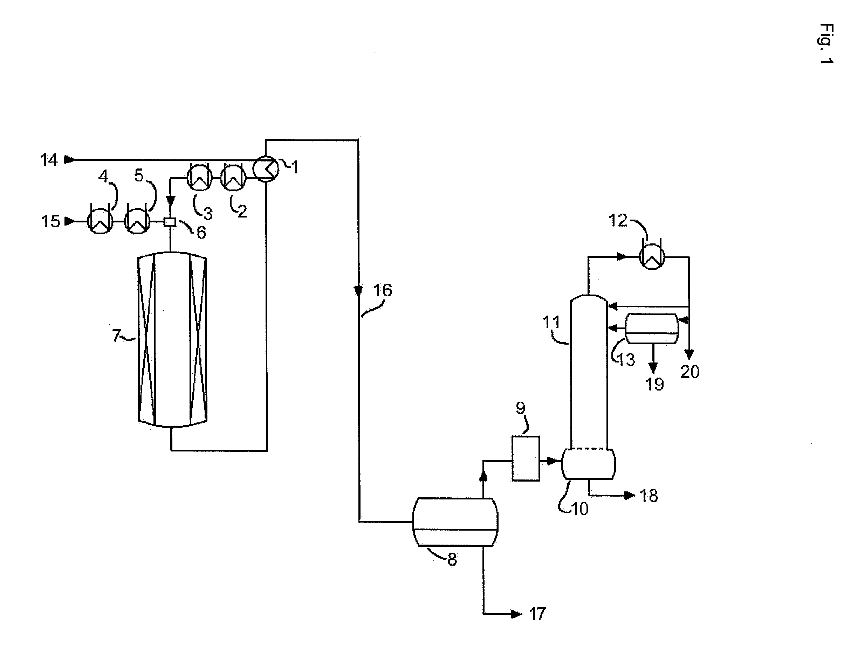

Typically, at the beginning of the process of the invention, THFA and water are fed separately through different lines for vaporization. At industrial scale, conveniently, waste heat may be used for heating the educts. In case waste heat is not sufficient to heat the educts to the desired temperature, additional vaporizers may be used. Typically, the vaporized THFA is entrained in the water vapour immediately before entering the reactor in a mixing device (see Figure 1 and Example below).

The weight ratio between THFA and water in the vapour phase when contacting the Al₂O₃ comprising catalyst is typically of from 1/10 to 10/1, preferably of from 1/5 to 5/1, and more preferably of from 1/2 to 2/1, for example 1/1.

Typically, the THFA is contacted with the solid phase catalyst by passing the THFA entrained in the carrier gas over/through the catalyst. In the catalyst used according to the invention, the Al₂O₃ may be used as the sole catalyst component, or Al₂O₃ may be used in combination with one or more further catalytically active components. Examples of catalytically active components include further oxides such as TiO₂, V₂O₅ or MoO₃. The content of Al₂O₃ in the solid phase catalyst typically is within a range of from 50 to 100 weight % and preferably is at least 75 weight %, more preferably at least 90 weight %. Most preferably, the catalyst is essentially composed of Al₂O₃ Preferably, the η-modification of Al₂O₃ is used (η-Al₂O₃).

The THFA entrained in the water vapour typically is continuously passed through a heated reactor containing the Al₂O₃ catalyst. The residence time in the reactor is typically of from 0.5 to 20 sec, preferably of from 1 to 10 sec, more preferably of from 1 to 5 sec, and most preferably of from 1 to 2 sec.

The process of the present invention, i.e. conversion of THFA on the Al₂O₃ comprising catalyst, is typically carried out at a temperature of from 280 to 380 °C, preferably of from 300 to 350 °C, for example at 330 °C.

According to the present invention, a small amount of nitrogen may be optionally used in the conversion reaction. Nitrogen may be used for safety reasons and for maintaining a stable educt feed. The amount of nitrogen is typically of from 0.1 to 3 weight %, preferably of from 0.5 to 2 weight % relative to the THFA/water vapour mixture passed over the catalyst.

In the reactor, the THFA vapour is converted to DHP vapour and water vapour. The obtained DHP and water vapours, including the water vapour of the carrier gas, leaving the reactor are condensed and are typically passed through a separator or decanter where the water is removed from DHP. It must be noted that the water vapour in the product stream has not the same significance and effect then the water vapour used as an entrainer gas, which leads all chemicals in the desired direction (i.e. towards the exit of the tube reactor). Water vapour produced in the process as such has no flow direction at all.

As can be seen from Figures 2 to 7, the conversion rate of THFA to DHP was consistently quantitative over four days. The amounts of generated by-products were low and did not increase over time. This demonstrates stable conversion with excellent yields when using water vapour as carrier gas. It is also evident that the process of the present invention may be carried out over long periods of time before regeneration of the catalyst is required. In the state of the art the life time of the catalyst in production corresponds to the recycle time of the catalyst. Under the conditions of the present invention even after 4.5 days of production the catalyst revealed no tendency of inactivation. Also, after 4.5 days by optical control the catalyst looked like fresh prepared catalyst and no deterioration of the catalyst could be observed.

Except for the start-up phase, the content of DHP in the crude product turned out to be very high and at least 94 weight % when using water vapour as carrier gas. On the other hand, the amounts of the by-products THP, cyclopentanone and acrolein in the crude product are surprisingly low. Typically, the amount of THP is below 2.0 weight %, the amount of acrolein is below 0.05 weight % and the amount of cyclopentanone is below 0.1 weight %. Further, the amount of unreacted THFA is typically below 1.5 weight % and the amount of residual water is typically below 1.0 weight %. Specifically, the amounts of cyclopentanone and acrolein are considerably lower than in industrial processes using nitrogen as carrier gas. Using water vapour as entrainer also leads to unexpectedly low content of acrolein in the DHP product.

The DHP obtained according to the invention may be subjected to further purification, to meet the standards required for the production of pharmaceutical compounds or agro chemicals. The most common by-products generated in DHP production are THP, cyclopentanone and acrolein. Also some unreacted THFA is typically contained in the crude product. Further purification steps typically include distillation.

As THP, acrolein and cyclopentanone form azeotropic mixtures with the residual water in the crude product, the by-products may be removed by azeotropic distillation. Azeotropic distillation may be carried out according to methods known in the art.

The distillation may be carried out in continuous mode or in batch mode. In continuous mode the crude product leaving the separator is passed continuously into the distillation boiler, whereas in batch mode the crude product is first collected for example in a tank and then passed into the distillation boiler.

For more efficient separation, the distillation is typically carried out by rectification. To remove the azeotropic mixtures, the reflux may be passed partly or completely through a decanter filled with water, where the azeotropic mixtures and the contaminants accumulate.

The use of water vapour as carrier gas in the catalyzed production of DHP has the advantage that water is abundantly available and sufficient capacity for its vaporization (waste heat) is on hand in industrial facilities. Moreover, after completion of the reaction, the water may be easily condensed and separated from the crude DHP. The easy condensation and separation of the water also circumvents the problem of product loss. As the condensed water and the crude organic DHP are both in the liquid phase, their separation in a decanter is straightforward. While the disposed water may still contain some DHP, the DHP loss is less than when nitrogen gas is used which entrains a considerable amount of product.

Moreover, use of water vapour as a carrier gas requires few regeneration steps of the Al₂O₃ catalyst. Without wishing to be bound by theory, it is believed that running the catalytic conversion with water vapour avoids catalyst regeneration to a large extent, as steam has similar effects as air and oxygen. The catalyst is thus continuously regenerated in the presence of water vapour. To run a DHP production truly continuous in the art it is recommended to have two catalytic reactor columns in parallel, thus, while one reactor is in use, the catalyst in the other reactor can be recycled. The present invention allows operating with only one reactor to be truly continuous. Furthermore, no effluent products (organics, tar, carbon black...) from regenerating the catalyst need to be released to the atmosphere without regenerating with an oxygen containing gas. A further advantage of the "continuous regeneration" is that there is no induction phase while restarting the reaction after regeneration.

The present invention is illustrated in more detail by the following non-limiting examples.

### Example

### Production of DHP using H₂O vapour as carrier gas

In this specific embodiment, the conversion of THFA to DHP was carried out in continuous mode and run for four days. With reference to Fig. 1, the water (14) was pumped through heat exchanger (1) and heated by the raw product stream leaving the reactor. After leaving the heat exchanger (1) the water had a temperature of about 195 to 210 °C. The water vapour was further passed through vaporizers (2) and (3) to reach a temperature of about 370 to 395 °C.

5.3 tons of THFA (15) were pumped through heat exchanger (4) and then through vaporizer (5) to reach a temperature of 300 °C. The gaseous THFA was entrained in the water vapour in a mixing device (6) and passed through reactor (7) charged with 49.3 kg of Al₂O₃ (NorPro Saint-Gobain, SA 3X77). The temperature in the reactor was 350 °C at the beginning of the production run and was further maintained at 330 °C. The reactor pressure was maintained at atmospheric pressure. A small amount of nitrogen (1 kg/h) was fed into the system between heat exchanger (1) and vaporizer (2). The feed of the THFA was 60 kg/h and the feed of the water was about 54 kg/h respectively.

The residence time of the heated mixture in the reactor was approx. 1.3 sec and the WHSV (weight hourly space velocity) was 2.3 h⁻¹.

The obtained crude product was cooled when contacted with the feed water at heat exchanger (1) and nitrogen and other uncondensable gases were removed in a separator (not shown). The crude product (16), mainly consisting of DHP and water, was further passed into the decanter (8) and separated from water. The water was disposed (17) as it contained only about 1.6 weight % of DHP. Following separation from water, the crude product contained 94 weight % of DHP and the yield was 82 %.

The thus obtained crude product contained of 94 weight % of DHP, 1.8 weight % of THP, 1 weight % of THFA, 0.6 weight % of H₂O, 0.035 weight % of acrolein and 0.06 weight % of cyclopentanone.

An azeotropic distillation was carried out in batch mode. About 4.2 m³ of crude product collected in one or more tanks (9) after separation of water in the decanter were subjected to distillation. The crude product was filled into a 3.2 m³ distillation boiler (10) and heated to reflux over a column (11) with a structured packing (Sulzer Mellapack 250 Y) having 11 theoretical plates. The distillation was carried out at atmospheric pressure. The sump (18) was periodically removed.

At the beginning, the reflux was returned completely to the column with a rate of 900 l/h after passing a condenser (12). The decanter (13) was charged 2/3 with water, and 2/3 of the reflux was passed through the decanter before returning it to the column. The azeotropes remained in the decanter and the water (19) in the decanter was replaced every 30 min until the organic phase met the required specifications, particularly until the acrolein content was 20 ppm or less. At that point, the reflux ratio was set to 3 (reflux = 600 l/h and DHP removal = 200 l/h) and the DHP (20) was distilled into a suitable tank. The distillation was interrupted when the temperature in the distillation boiler reached 130 °C. The purified DHP thus obtained consisted of 97.77 weight % of DHP, 1.64 weight % of THP, 0.2 weight % of H₂O and 0.0017 weight % of acrolein as determined by gas chromatography. Cyclopentanone was below detection limits after distillation. DHP yield after distillation was 74 %. It must be noted that in some distillation batches we obtained a very low acrolein level of only 6 to 9 ppm.

## Claims

1. Process for producing 2,3-dihydropyran (DHP), comprising contacting tetrahydrofurfuryl alcohol (THFA) entrained in a carrier gas with a catalyst comprising aluminium oxide (Al₂O₃), wherein the carrier gas is water vapour.

2. Process according to claim 1, wherein the catalyst is continuously regenerated in the presence of the water vapour.

3. Process according to claim 1 or 2, wherein the weight ratio between THFA and water is from 1/5 to 5/1, preferably of from 1/2 to 2/1.

4. Process according to any of claims 1 to 3, wherein the catalyst further comprises a catalytically active oxide selected from the group consisting of TiO₂, V₂O₅ and MoO₃.

5. Process according to any of claims 1 to 4, wherein the process is carried out at a temperature of from 280 to 380 °C, preferably of from 300 to 350 °C.

6. Process according to any of claims 1 to 5, wherein the obtained dihydropyran is further subjected to a purification step, preferably to distillation.
